# EUROPÄISCHE PATENTSCHRIFT

(11) **EP 3 512 490 B1**
(45) Veröffentlichungstag und Bekanntmachung des Hinweises auf die Patenterteilung: **23.02.2022**
(21) Anmeldenummer: 17758903.3
(22) Anmeldetag: 05.09.2017
(51) Int. Cl.: A61K 8/65, A61K 38/01, A61Q 5/00, A61Q 5/12, A61Q 19/10

(54) **NIEDERMOLEKULARE KERATINHYDROLYSATE**
LOW MOLECULAR WEIGHT HYDROLYSATES OF KERATINE
KÉRATINE HYDROLYSÉ À MASSE MOLECULAIRE REDUITE

(30) Priorität: 13.09.2016 EP 16188513
(43) Veröffentlichungstag der Anmeldung: 24.07.2019
(73) Patentinhaber: BASF SE, 67056 Ludwigshafen am Rhein (DE)
(72) Erfinder: KOHLMANN, Christina, 40789 Monheim (DE); MARKIEFKA, Christian, 40789 Monheim (DE); PELLON, Guadalupe, 40789 Monheim (DE); HAAKE, Hans-Martin, 40589 Düsseldorf-Holthausen (DE); BEHLER, Ansgar, 40589 Düsseldorf-Holthausen (DE)
(74) Vertreter: BASF IP Association
(86) Internationale Anmeldenummer: PCT/EP2017/072219
(87) Internationale Veröffentlichungsnummer: WO 2018/050483

(56) Entgegenhaltungen:
- WO-A1-96/25141
- WO-A1-2015/086004
- WO-A1-2015/185340
- DE-A1-102007 031 202
- TATSUNOBU MATSUI ET AL: "Sustainable and practical degradation of intact chicken feathers by cultivating a newly isolated thermophilic Meiothermus ruber H328", APPLIED MICROBIOLOGY AND BIOTECHNOLOGY, SPRINGER, BERLIN, DE, Bd. 82, Nr. 5, 5. Februar 2009 (2009-02-05), Seiten 941-950, XP019705524, ISSN: 1432-0614

## Beschreibung

### Gebiet der Erfindung

Die Erfindung liegt auf dem Gebiet der Kosmetik und betrifft niedermolekulare Keratinhydrolysate, Verfahren zu deren Herstellung und deren Verwendung als auch Haar- und Hautbehandlungsmittel.

### Stand der Technik

Proteine und insbesondere Keratin sind wichtige Inhaltstoffe der kosmetischen Industrie. Aufgrund ihrer filmbildenden Eigenschaften können Proteine eine schützende Schicht auf Haut und Haar ausbilden und sie somit pflegen. Proteine haben eine Vielzahl weiterer Vorteile und finden daher breite Anwendung in kosmetischen Mitteln für Haut und Haar; wobei Keratin vornehmlich in der Haarpflege eingesetzt wird.

Aufgrund ihrer schlechten Löslichkeit werden die Proteine jedoch meist nicht in ihrer natürlichen Form, sondern als sogenannte "Hydrolysate" in kosmetischen Formulierungen eingesetzt. Bei der Hydrolyse werden die Peptidbrücken innerhalb eines Proteins durch die Einwirkung von starken Säuren, Basen oder Katalysatoren aufgebrochen und man erhält Proteinhydrolysate, also Mischungen aus kleineren Fragmenten wie Peptiden und unter Umständen sogar Aminosäuren. Je nach Art der Herstellmethode weisen die Proteinhydrolysate andere Proteinbruchstücke auf. Da die chemische Hydrolyse mittels Säuren oder Basen unspezifisch abläuft und auch häufig zu Produkten mit schlechteren Qualitäten, insbesondere in Hinblick auf Farbe, Geruch und Stabilität führt, ist die enzymatische Hydrolyse der Proteine besonders für kosmetische Anwendungen die bevorzugte Methode. Im Gegensatz zur chemischen Hydrolyse läuft die enzymatische Hydrolyse bei moderaten Reaktionsbedingungen hinsichtlich pH-Wert, Temperatur und Druck ab. Des Weiteren sind die eingesetzten Enzyme für ihre Spezifität bekannt, d.h. im Gegensatz zur chemischen Hydrolyse ist die Zusammensetzung der sich ergebenden Proteinhydrolysate einheitlicher und führt so zu gleichbleibenden Produktzusammensetzungen.

Keratinhydrolysate können aus den natürlichen Rohstoffquellen wie Wolle, Haare, Federn, Huf oder Horn durch chemische und/oder enzymatische Hydrolyse hergestellt werden. So wird beispielsweise in der Deutschen Auslegeschrift DE 2705669 ein enzymatischer Abbau von Haaren, Wolle oder dergleichen beschrieben, wobei zuerst eine klassische chemische Säurehydrolyse erfolgt und anschließend im alkalischen die enzymatische Hydrolyse mittels Proteinasen.

Als alkalische Proteinasen werden in der genannten Auslegeschrift solche aus Bacillusstämmen empfohlen, wie Bacillus licheniformis, Bacillus alcaophilus, Bacillus subtilis, Bacillus mesentericus oder Bacillus firmus verwendet.

Die genannten Proteinasen sind auch unter den Begriffen "Endoproteasen" oder "Endopeptidasen" bekannt und spalten die Proteinbindung im Inneren des Proteins.

Aus der Internationalen Patentanmeldung WO 96/25141 A1 werden kationisierte pflanzliche Proteintenside beschrieben, die zunächst bei einem pH-Wert im Bereich von 8 bis 10 mit Proteinasen und ggf. anschließend bei einem pH-Wert im Bereich von 6-7 mit (Exo)Peptidasen hydrolysiert werden, bevor diese mit quartären Ammoniumsalzen zu den kationisch modifizierten Produkten umgesetzt werden, die als Tenside in der Kosmetik verwendbar sind.

In der Internationalen Patentanmeldung WO 2008/115165 A2 werden ganz speziell hydrolysierte Reisproteine beschrieben, wobei zunächst mit der Endoprotease Alcalase^{®} im alkalischen bei pH 7,5 bis 8 und anschließend mit einer Protease wie Flavorzyme^{®} bei pH 6,5 eine enzymatische Hydrolyse erfolgt. Dem schließt sich eine weitere Hydrolyse mit dem Dual Protease Enzyme^{®} und Neutrase (Novo) an, bevor mit einem Membranfilter filtriert wird. Das Retenat hat ein durchschnittliches Molekulargewicht von 3500 und das Permeat von 200.

Die Deutsche Patentanmeldung DE 10 2007 031 202 A1 beschreibt Hydrolysate aus Vikunja-Wolle.

So erhaltene Proteinhydrolysate vermögen an der Haaroberfläche zu adsorbieren und dem Haar zu mehr Substantivität zu verhelfen. Die Schuppenschicht des Haars erscheint glatter und das Haar wirkt gesünder und glänzender. Mit anderen Worten, die Schutzschicht des Haars wird von außen gestärkt, aber nicht von innen, d.h. das Haar wird nicht elastischer.

Aufgabe der vorliegenden Erfindung war es, Keratinhydrolysate bereitzustellen, deren Anwendung in Haarpflegeprodukten zu einer Stärkung des Haares von innen führt, also zur Verbesserung der inneren Haarstruktur beiträgt. Die Haare sollten elastischer bleiben bzw. vorgeschädigtes Haar sollte mittels den bereitgestellten Keratinhydrolysaten sich von innen regenerieren bzw. reparieren, so dass eine höhere Elastizität (wieder)gewonnen wird. Zudem sollten die Keratinhydrolysate eine sehr gute Konditionerwirkung zeigen. Unter Haarkonditionierung versteht der Fachmann die Behandlung von Haaren mit pflegenden so genannten Rinse-off-Formulierungen (also Formulierungen, die abgespült werden) oder so genannten Leave-on-Formulierungen (also Formulierungen, die auf den Haaren verbleiben, ohne abgespült zu werden), insbesondere mit pflegenden Shampoos oder Spülungen (engl. Conditioner). Diese Behandlung sollte insbesondere eine leichtere Kämmbarkeit der Haare im nassen und trockenen Zustand, sowohl in den Längen als auch in den Spitzen (bessere Entwirrbarkeit), zu verbesserten taktilen Eigenschaften wie Glätte, Weichheit und Geschmeidigkeit sowie zu mehr Haarglanz, weniger elektrostatischer Aufladung und besserer Frisierbarkeit bewirken. Insgesamt sollte mit der Behandlung mit einem Conditioner ein gepflegter und gesund wirkender Gesamtzustand des Haares erzielt werden.

Schließlich sollten die Keratinhydrolysate leicht herstellbar, sehr lagerstabil ohne Verdickung und leicht formulierbar in Haar- und Hautbehandlungsmitteln sein.

Die Aufgabe wurde gelöst durch Keratinhydrolysate, dadurch gekennzeichnet, dass mindestens 30 % der enthaltenen Proteinfragmente ein Molekulargewicht unter 1000 Da und mindestens 15 % ein Molekulargewicht unter 500 Da aufweisen.

Ein erster Gegenstand der vorliegenden Erfindung ist das Verfahren zur Herstellung von Keratinhydrolysaten, bei denen mindestens 30 % der enthaltenen Proteinfragmente ein Molekulargewicht unter 500 Da und mindestens 15% ein Molekulargewicht unter 500 Da aufweisen, wobei zunächst a) Keratin oxidativ vorbehandelt und b) in Anwesenheit mindestens einer Endopeptidase enzymatisch hydrolysiert wird, dadurch gekennzeichnet, dass anschließend c) in Anwesenheit von mindestens einer Exopeptidase ein weiteres Mal enzymatisch hydrolysiert wird.

Weitere Gegenstände der Erfindung betreffen die Verwendung der Proteinhydrolysate zur Verbesserung mindestens einer der Eigenschaften in Haarbehandlungsmitteln:
- Verbesserung der inneren Haarstruktur
- Schutz vor haarschädigenden Umwelteinflüssen
- Reparatur geschädigter Haare
- Zugfestigkeit von menschlichen Haaren
- Stabilisierung des Feuchtigkeitshaushaltes von menschlichen Haaren
- Kämmbarkeit von menschlichen Haaren
- Haarbruch von menschlichen Haaren
- Restrukturierbarkeit von menschlichen Haaren
- Verringerung der Elastizitätsabnahme von menschlichen Haaren.

Auch die Verwendung der Keratinhydrolysate als Conditioner in Haarbehandlungsmitteln und deren Verwendung zur Verbesserung der Sensorik in kosmetischen Mitteln zur Reinigung von Haut und/oder Haar, vorzugsweise der Schaumsensorik und insbesondere in Duschbädern oder Duschgelen, sind Gegenstände der vorliegenden Erfindung sowie kosmetische Mittel enthaltend die erfindungsgemäßen Keratinhydrolysate in Mengen von 0,01 bis 2 Gew.-% einer 20 gew.-% igen Keratinhydrolysat-Lösung bzw. 0,002 bis 0,4 Gew.-% bezogen auf Aktivsubstanzgehalt an Keratinhydrolysat.

Im Sinne der vorliegenden Erfindung ist das Molekulargewicht in Dalton (Da) definiert und die Molekulargewichtsverteilung wurde mittels Flüssigchromatografie bestimmt.

Zur Bestimmung der Molekulargewichtsverteilung wurde das Flüssigchromatographiegerät Agilent 1260 Infinity mit binärer Pumpe und Entgaser in Kombination mit PSS WinGPC UniChrom verwendet. PSS WinGPC UniChrom ist ein makromolekulares Chromatographie Daten System mit herstellerunabhängiger Echtzeit-Datenerfassung zur umfassenden Analytik von Makromolekülen und besonders geeignet für die Analyse von Polymeren, Biopolymeren und Proteinen. Bei der verwendeten Chromatographiesäule handelt es sich um eine spezielle Chromatographiesäule ("Superdex Peptide 10/300 GL" von GE Healthcare Life Science, Porenweite 100 Ä, 5 µm Partikelgröße) zur hochauflösenden Trennung von Proteinen und Peptiden. Diese Säule wurde ausgewählt, da sie besonders gut geeignet ist um Biomoleküle mit Molekulargewichten zwischen 100 und 7000 zu bestimmen. Als Laufmittel wurde verdünnte Salzsäure (0,05 M) mit einer Fließgeschwindigkeit von 0,5 mL/min eingesetzt. Die Detektion erfolgte mit einem Dioden-Array-Detektor (DAD) bei 214 nm.

Im Sinne der vorliegenden Erfindung ist der Begriff der Proteinfragmente definiert als eine Mischung aus Bruchstücken erhalten aus dem Abbau des ursprünglichen Proteins Keratin. Derartige Mischungen enthalten freie Aminosäuren und peptidisch gebundene Aminosäuren. Es ist im Rahmen der Erfindung wesentlich, dass hohe Anteile von mindestens 30 % der enthaltenen Proteinfragmente ein als niedermolekular zu bezeichnendes Molekulargewicht unter 1000 Da und mindestens 15 % ein Molekulargewicht unter 500 Da aufweisen. Bevorzugt im Sinne der Erfindung enthalten die Keratinhydrolysate mindestens 35 % Proteinfragmente mit einem Molekulargewicht unter 1000. Im Sinne der Erfindung sind die Proteinfragmente Mischungen, die neben den freien Aminosäuren auch peptidisch gebundene Aminosäuren enthalten. Vorzugsweise haben daher mindestens 50 % der enthaltenen Proteinfragmente mit einem Molekulargewicht unter 1000 Da ein Molekulargewicht über 100 Da.

Erfindungsgemäß sind Keratinhydrolysate, wobei 15 % der enthaltenen Proteinfragmente ein Molekulargewicht unter 500 Da aufweisen und insbesondere solche, bei denen von diesen wiederum mindestens 50 % ein Molekulargewicht über 100 Da aufweisen.

Keratinhydrolysate mit derartig hohen Anteilen an niedermolekularen Proteinfragmenten sind gemäß dem erfindungsgemäßen mehrstufigen Verfahren zugänglich.

Als Ausgangsmaterial können keratinhaltige Abfallprodukte wie Haare oder Wolle von Tieren, insbesondere Schafswolle eingesetzt werden.

In der Vorbehandlung a) wird das eingesetzte Keratin oxidativ vorbehandelt, wobei das keratinhaltige Ausgangsmaterial vorzugsweise in eine wäßrige Suspension überführt wird, die mittels einem Oxidationsmittel im alkalischem Milieu mehrere Stunden vorbehandelt wird. Bevorzugt wird das Keratin in Form einer wäßrigen Suspension mittels Wasserstoffperoxid bei pH-Werten zwischen 8 bis 12 mindestens 8 Stunden oxidativ vorbehandelt und insbesondere bei einem pH-Wert von 9 bis 11 und etwa 9 bis 11 Stunden.

Im Verfahrensschritt b) wird mittels mindestens einer Endopeptidase enzymatisch hydrolysiert, vorzugsweise bei einem alkalischen pH-Wert im Bereich von 8 bis 9. Bevorzugt sind die Endopeptidasen ausgewählt aus den Bacillusstämmen, insbesondere Bacillus licheniformis, Bacillus alcaophilus, Bacillus subtilis, Bacillus mesentericus oder Bacillus firmus. Insbesondere geeignet sind solche aus Bacillus licheniformis, die im Handel erhältlich sind beispielweise als Alcalase^{®} erhältlich bei Novozymes.

Im Verfahrensschritt c) wird in Anwesenheit von mindestens einer Exopeptidase ein weiteres Mal enzymatisch hydrolysiert, vorzugsweise bei einem neutralen pH-Wert von 6 bis 8. Als Exopeptidase zählen die alpha-Aminoacylpeptid-Hydrolasen (EC 3.4.11), die am Ende des Polypeptids einzelne Aminosäuren ablösen, die Dipeptid-Hydrolasen bzw. Dipeptidasen (EC 3.4.13), die Dipeptide zu Aminosäuren hydrolysieren, die Dipeptidylpeptid-Hydrolasen bzw. Dipeptidylpeptidasen (EC 3.4.14), die Aminoständige Dipeptide eines Polypeptids freisetzen, Peptidyldipeptid-Hydrolasen bzw. Dipeptidylcarboxypeptidasen (EC 3.4.15), die einzelne Aminosäuren des Carboxy-Terminus abtrennen, Carboxypeptidasen (EC 3.4.16 - 3.4.18) und Omega-Peptidasen (EC 3.4.19), die modifizierte Aminosäuren von beiden Ende des Polypeptids abspalten. Insbesondere bevorzugt sind solche zugänglich aus Aspergillus oryzae wie das im Handel erhältliche Flavourzyme^{®} von Novozymes.

Die enzymatische Hydrolysen b) und c) erfolgen vorzugsweise bis zur Vollständigkeit, die für den Fachmann in bekannter Art und Weise bestimmbar ist, beispielsweise durch Ermittlung des konstanten pH-Wertes oder mittels Größenausschlusschromatographie oder photometrisch mittels Nachweis freier NH₂-Gruppen.

Die Menge der eingesetzten Endopeptidase bzw. Exopeptidase ist an sich nicht kritisch, sollte jedoch im Bereich von 0,05 bis 5, vorzugsweise 0,1 bis 2 Gew.-% - bezogen auf keratinhaltigen Ausgangsstoff - liegen.

Die erhaltenen Keratinhydrolysate können abschließend aufgearbeitet werden, beispielsweise durch Abfiltration nichtgelöster Anteile. Vorzugsweise wird der pH-Wert zur besseren Stabilisierung auf Werte zwischen 3,5 und 5 eingestellt.

Die erhaltenen Keratinhydrolysate sind wässrige Lösungen und können direkt oder in aufkonzentrierter Form eingesetzt werden, vorzugsweise weisen sie einen Feststoffgehalt im Bereich von 10 bis 50 Gew.-%, vorzugsweise 15 bis 30 Gew.-% auf. Es ist aber auch möglich, durch Entwässerung die Keratinhydrolysate als Pulver zu konfektionieren.

Nach einer Verfahrensvariante erfolgen die Verfahrensschritte a), b) und c) hintereinander entsprechend Verfahrensanspruch 1. Nach einer anderen Verfahrensvariante setzt man auf dem Markt handelsübliches Keratinhydrolysat ein und hydrolysiert enzymatisch ein weiteres Mal analog dem schon beschriebenen Verfahrensschritt c).

Einer weiteren Ausführungsform der Erfindung entsprechend werden die erfindungsgemäßen Keratinhydrolysate zur Verbesserung mindestens einer der Eigenschaften in Haarbehandlungsmitteln:
- Verbesserung der inneren Haarstruktur
- Schutz vor haarschädigenden Umwelteinflüssen
- Reparatur geschädigter Haare
- Zugfestigkeit von menschlichen Haaren
- Stabilisierung des Feuchtigkeitshaushaltes von menschlichen Haaren
- Kämmbarkeit von menschlichen Haaren
- Haarbruch von menschlichen Haaren
- Restrukturierbarkeit von menschlichen Haaren
- Verringerung der Elastizitätsabnahme von menschlichen Haaren
verwendet.

Besonders vorteilhaft ist die Verwendung zur Verbesserung der Zugfestigkeit der menschlichen Haare, die Verringerung der Elastizitätsabnahme von menschlichen Haaren sowie deutlich weniger Haarbruch.

Unter Haarbehandlungsmittel sind alle kosmetischen Haarbehandlungsmittel zu verstehen, die zur Reinigung, Pflege, Trocknung, Farbänderung oder Strukturänderung des Haares gedacht sind. Beispielsweise sind darunter Haarshampoos, Haarkonditionierer, konditionierende Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fön-Lotionen, Schaumfestiger, Haargele, Haarwachse oder Kombinationen davon zu verstehen.

Die erfindungsgemäßen Keratinhydrolysate können erfindungsgemäß als Conditioner in Haarbehandlungsmittel verwendet, insbesondere in pflegenden Haarshampoos, Haarkonditionierer, konditionierenden Shampoos, Haarspülungen, Haarkuren, Haarpackungen, Haarseren, Haartonik, Haarfestiger, Haarstyling-Zubereitungen, Schaumfestiger, Haargele und/oder Haarwachse.

In den Haarbehandlungsmitteln sind typischerweise weitere Inhaltsstoffe wie Tenside, Emulgatoren, Co-Tenside, (kationische) Polymere, Ölkörper, Emulgatoren, Perlglanzwachse, Konsistenzgeber, Verdickungsmittel, Überfettungsmittel, Stabilisatoren, Siliconverbindungen, Fette, Wachse, Lecithine, Phospholipide, UV-Lichtschutzfaktoren, biogene Wirkstoffe, Antioxidantien, Antischuppenmittel, Filmbildner, Quellmittel, Tyrosininhibitoren (Depigmentierungsmittel), Hydrotrope, Solubilisatoren, Konservierungsmittel, Parfümöle, Farbstoffe und dergleichen enthalten.

Ein weiterer Gegenstand der vorliegenden Erfindung betrifft die Verwendung der erfindungsgemäßen Keratinhydrolysate zur Verbesserung der Sensorik in kosmetischen Mitteln zur Reinigung von Haut und/oder Haar, vorzugsweise der Schaumsensorik und insbesondere in Duschbädern oder Duschgelen.

In den kosmetischen Mitteln zur Reinigung von Haut und/oder Haar sind typischerweise als weitere Inhaltsstoffe die bereits unter den Haarbehandlungsmitteln aufgezählten Inhaltsstoffe wie Tenside, Emulgatoren, Co-Tenside, (kationische) Polymere, Ölkörper, Emulgatoren, Perlglanzwachse, Konsistenzgeber, Verdickungsmittel, Überfettungsmittel, Stabilisatoren, Fette, Wachse, Lecithine, Phospholipide, biogene Wirkstoffe, Antioxidantien, Antischuppenmittel, Filmbildner, (Quellmittel), Hydrotrope, Solubilisatoren, Konservierungsmittel, Parfümöle, Farbstoffe und dergleichen enthalten.

In den kosmetischen Mitteln sind die erfindungsgemäßen Keratinhydrolysate in Mengen von 0,002 bis 0,4, vorzugsweise in Mengen von 0,01 bis 0,4 Gew.-% - bezogen auf Aktivsubstanzgehalt - enthalten.

Im Folgenden werden geeignete weitere Inhaltsstoffe für die erfindungsgemäße Verwendung in den kosmetischen Mitteln aufgeführt.

### Tenside

Als oberflächenaktive Stoffe können anionische, nichtionische, kationische, amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Alk(en)ylpolyglykolethercitraten bzw. deren Salzen, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, partiell oxidierte Alk(en)yloligoglykoside bzw. Glucoronsäurederivate, Fettsäure-N-alkylglucamide, Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für kationische Tenside sind quartäre Ammoniumverbindungen, wie beispielsweise das Dimethyldistearylammoniumchlorid sind insbesondere quartäre Ammoniumverbindungen verwendbar. Bevorzugt sind Ammoniumhalogenide, insbesondere Chloride und Bromide, wie Alkyltrimethylammiumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im Allgemeinen quaternierte Fettsäuretriethanolaminestersalze verstanden. Besonders bevorzugt sind als kationische Tenside Produkte, die unter als Dehyquart^{®} L80, Dehyquart^{®} F 75, Dehyquart^{®} A-CA bekannt sind.

Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Fettsäureglucamide und/oder Proteinfettsäurekondensate. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen.

Unter den nichtionischen Tensiden werden insbesondere für kosmetische Hautbehandlungsmittel wie Duschgele und Duschbäder Alkyl- und Alkenyloligoglykoside bevorzugt, die der Formel

**RO-[G]ₚ**

folgen, in der R für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldi-methylamin sowie deren technische Gemische. Weiterhin kommen auch Carboxyalkylierungsprodukte von Amidoaminen in Betracht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethyla-minoethylamin, N,N-Dimethylaminopropylamin, N,N-Diethylaminoethylamin und N,N-Diethylami-nopropylamin, die mit Natriumchloracetat kondensiert werden. Bevorzugt ist der Einsatz eines Kondensationsproduktes von C_{8/18}-Kokosfettsäure-N, N-dimethylaminopropylamid mit Natriumchloracetat.

Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, E-rucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Silikonöle (cyclische Dimethicone sog. (INCI) Cyclomethicone, Polymethylsiloxane, sog.(INCI) Dimethicone , aminofunktionelle Silikone, sog. (INCI) Amodimethicone wie Trimethylsilylamodimethicone u.a.) und/oder aliphatische bzw. naphthenische Kohlenwasserstoffe, wie z.B. wie Squalan, Squalen oder Dialkylcyclohexane in Betracht. Geeignete Silikonöle werden in dem Europäischen Patent EP1830798 auf den Seiten 8-14 beschrieben, auf die hier ausdrücklich Bezug genommen wird.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Addukte mit 1 bis 30 Mol Ethylenoxid an Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen;
- Partialester von Polyglycerin (durchschnittlicher Eigenkondensationsgrad 2 bis 8), Polyethylenglycol (Molekulargewicht 400 bis 5000), Sorbitan, Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

Besonders bevorzugte Emulgatoren sind Anlagerungsprodukte von Ethylenoxid an C_{12/18}-Fett-säuremono- und -diester, Anlagerungsprodukte von 1 bis 30, vorzugsweise 5 bis 10 Mol Ethylenoxid an Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische. Ebenfalls geeignet sind Anlagerungsprodukte von 1 bis 30, vorzugsweise 5 bis 10 Mol Ethylenoxid an Sorbitanester. Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische in Frage. Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} Gl 34), Polyglyceryl-3 Oleate, Diisostearoyl Polyglyceryl-3 Diisostearate (lsolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen. Bevorzugt ist auch Trimethylpropan-EO/PO-Trioleat, ein Gemisch erhältlich durch die Umsetzung von Trimethylolpropantrioleat mit Ethylenoxid und Propylenoxid unter alkalischen Bedingungen. Dabei findet, zumindest zum Teil, eine Einlagerung der Ethylenoxideinheiten (EO) und der Propylenoxideinheiten (PO) in die Estergruppen des Trimethylolpropantrioleat statt. Das Trimethylpropan-EO/PO-Trioleat wird charakterisiert durch seinen Gehalt an EO- und PO-Einheiten pro Molekül im statistischen Mittel. In einer Ausführunsgform der vorliegenden Erfindung wird Trimethylpropan-EO/PO-Trioleat, mit 120 Ethylenoxideinheiten (EO) und 10 Propylenoxideinheiten (PO) eingesetzt.

### Wachse und Perlglanzwachse

Perlglanzwachse vermachen den kosmetischen Zubereitungen einen weiß-schimmernden, perlähnlichen Effekt, der besonders in Haarshampoos und Duschgels geschätzt wird. Es können aber auch Wachse ohne Perlglanzeffekt in den Haarbehandlungsmitteln enthalten sein.

Geeignete Wachskörper sind: Alkylenglycolester, Fettsäurealkanolamide, Partialglyceride, Ester von mehrwertigen, gegebenenfalls hydroxysubstituierten Carbonsäuren, Fettalkohole, Fettketone, Fettaldehyde, Fettether, Fettcarbonate, Ringöffnungsprodukte von Olefinepoxiden sowie deren Mischungen.

Bei den **Alkylenglycolestern,** handelt es sich üblicherweise um Mono- und/oder Diester von Alkylenglycolen, die der Formel (I) folgen,

**R¹CO(OA)ₙOR²** **(I)**

in der R¹CO für einen linearen oder verzweigten, gesättigten oder ungesättigten Acylrest mit 6 bis 22 Kohlenstoffatomen, R² für Wasserstoff oder R¹CO und A für einen linearen oder verzweigten Alkylenrest mit 2 bis 4 Kohlenstoffatomen und n für Zahlen von 1 bis 5 steht. Typische Beispiele sind Mono- und/oder Diester von Ethylenglycol, Propylenglycol, Diethylenglycol, Dipropylenglycol,Triethylenglycol oder Tetraethylenglycol mit Fettsäuren mit 6 bis 22, vorzugsweise 12 bis 18 Kohlenstoffatomen als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Ethylenglycolmono- und/oder -distearat. Andere Wachskörper wie **Fettsäurealkanolamide,** folgender Formel (II),

**R³CO-NR⁴-B-OH** **(II)**

in der R³CO für einen linearen oder verzweigten, gesättigten oder ungesättigten Acylrest mit 6 bis 22 Kohlenstoffatomen, R⁴ für Wasserstoff oder einen gegebenenfalls hydroxysubstituierten Alkylrest mit 1 bis 4 Kohlenstoffatomen und B für eine lineare oder verzweigte Alkylengruppe mit 1 bis 4 Kohlenstoffatomen steht. Typische Beispiele sind Kondensationsprodukte von Ethanolamin, Methylethanolamin, Diethanolamin, Propanolamin, Methylpropanolamin und Dipropanolamin sowie deren Mischungen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Stearinsäureethanolamid.

**Partialglyceride,** stellen Mono und/oder Diester des Glycerins mit linearen, gesättigten und/oder partiell ungesättigten Fettsäuren, beispielsweise Capronsäure, Caprylsäure, Caprinsäure, Laurinsäure, Myristinsäure, Palmitinsäure, Palmoleinsäure, Talgfettsäure, Stearinsäure, Behensäure sowie deren technische Mischungen dar. Sie folgen der Formel (III), 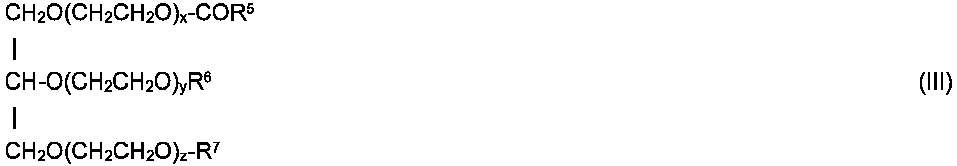 in der R⁵CO für einen Acylrest mit 6 bis 22 Kohlenstoffatomen, vorzugsweise für einen linearen, gesättigten Acylrest mit 6 bis 22 Kohlenstoffatomen, R⁶ und R⁷ unabhängig voneinander für Wasserstoff oder R⁵CO, x, y und z in Summe für 0 oder für Zahlen von 1 bis 30 und X für ein Alkali- oder Erdalkalimetall mit der Maßgabe steht, dass mindestens einer der beiden Reste R⁶ und R⁷ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Talgfettsäuremonoglycerid, Talgfettsäurediglycerid, Behensäuremonoglycerid, Behensäurediglycerid sowie deren technische Gemische, die untergeordnet aus dem Herstellungsprozeß noch geringe Mengen an Triglycerid enthalten können.

Als Wachskörper kommen weiterhin als bevorzugte Gruppe **Ester von mehrwertigen, gegebenenfalls hydroxysubstituierten Carbonsäuren** mit Fettalkoholen mit 6 bis 22 Kohlenstoffatomen in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bernsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll oder Partialester vorliegen, vorzugsweise werden Mono- und vor allem Diester der Carbon- bzw. Hydroxycarbonsäuren eingesetzt. Typische Beispiele sind Bernsteinsäuremono- und -dilaurylester, Bernsteinsäuremono- und -dicetearlyester, Bernsteinsäuremono- und -distearylester, Weinsäuremono- und -dilaurylester, Weinsäuremono- und dikokosalkylester, Weinsäuremono- und -dicetearylester, Citronensäuremono-, -di- und -trilaurylester, Citronensäuremono-, -di- und -trikokosalkylester sowie Citronensäuremono-, - di- und -tricetearylester.

Als dritte bevorzugte Gruppe von Wachskörpern können **Fettalkohole** eingesetzt werden, die der Formel (IV) folgen,

**R⁸OH** **(IV)**

in der R⁸ für einen linearen, gegebenenfalls hydroxysubstituierten Alkylrest und/oder Acylrest mit 16 bis 48, vorzugsweise 18 bis 36 Kohlenstoffatomen steht. Typische Beispiele für geeignete Alkohole sind Cetearylalkohol, Hydroxystearylalkohol, Behenylalkohol sowie Oxidationsprodukte langkettiger Paraffin.

**Fettketone,** die als Komponente in Betracht kommen, folgen vorzugsweise der Formel (V),

**R⁹-CO-R¹⁰** **(V)**

in der R⁹ und R¹⁰ unabhängig voneinander für Alkyl- und/oder Alkenylreste mit 1 bis 22 Kohlenstoffatomen stehen, mit der Maßgabe, dass sie in Summe mindestens 24 und vorzugsweise 32 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R¹³ und R¹⁴ aber nur um ein Kohlenstoffatom und leiten sich von Fettsäuren mit 16 bis 22 Kohlenstoffatomen ab.

Als Wachskörper geeignete **Fettaldehyde** entsprechen vorzugsweise der Formel (VI),

**R¹¹COH** **(VI)**

in der R¹¹CO für einen linearen oder verzweigten Acylrest mit 24 bis 48, vorzugsweise 28 bis 32 Kohlenstoffatomen steht.

Ebenso kommen **Fettether** vorzugsweise der Formel **(VII)** in Frage,

**R¹²-O-R¹³** **(VII)**

in der R¹² und R¹³ unabhängig voneinander für Alkyl- und/oder Alkenylreste mit 1 bis 22 Kohlenstoffatomen stehen, mit der Maßgabe, dass sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.

Als Komponente kommen weiterhin **Fettcarbonate** vorzugsweise der Formel **(VIII)** in Betracht,

**R¹⁴O-CO-OR¹⁵** **(VIII)**

in der R¹⁴ und R¹⁵ unabhängig voneinander für Alkyl- und/oder Alkenylreste mit 1 bis 22 Kohlenstoffatomen stehen, mit der Maßgabe, dass sie in Summe mindestens 24 und vorzugsweise 32 bis 48 Kohlenstoffatome aufweisen. Die Stoffe werden erhalten, indem man beispielsweise Dimethyl- oder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen R¹⁴ und R¹⁵ gleich sind und für Alkylreste mit 16 bis 22 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, Behenylalkohol und/oder Erucylalkohol in Form ihrer Mono- und Diester bzw. deren technischen Mischungen.

Bei den **Epoxidringöffnungsprodukten** handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel (IX), 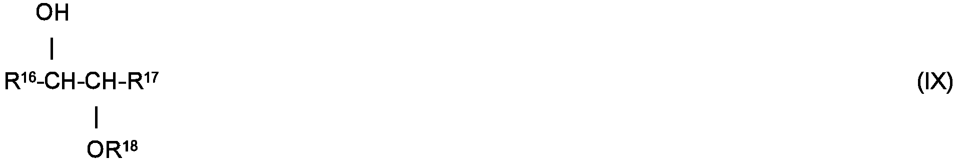 in der R16 und R17 für Wasserstoff oder einen Alkylrest mit 10 bis 20 Kohlenstoffatomen steht, mit der Maßgabe, dass die Summe der Kohlenstoffatome von R16 und R17 im Bereich von 10 bis 20 liegt und R18 für einen Alkyl- und/oder Alkenylrest mit 12 bis 22 Kohlenstoffatomen und/oder den Rest eines Polyols mit 2 bis 15 Kohlenstoffatomen und 2 bis 10 Hydroxylgruppen steht. Typische Beispiele sind Ringöffnungsprodukte von α-Dodecenepoxid, α-Hexadecenepoxid, α-Octadecenepoxid, α-Eicosen-epoxid, α-Docosenepoxid, i-Dodecenepoxid, i-Hexadecenepoxid, i-Octadecenepoxid, i-Eicosenepoxid und/ oder i-Docosenepoxid mit Laurylalkohol, Kokosfettalkohol, Myristylalkohol, Cetylalkohol, Cetearylalkohol, Stearylalkohol, Isostearylalkohol, Oleylalkohol, Elaidylalkohol, Petroselinylalkohol, Linolylalkohol, Linolenylalkohol, Behenylalkohol und/oder Erucylalkohol. Vorzugsweise werden Ringöffnungsprodukte von Hexa- und/oder Octadecenepoxiden mit Fettalkoholen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Die Anwesenheit mindestens eines kationischen Polymers ist vorteilhaft, vorzugsweise aus der Gruppe der kationisch modifizierten Cellulosederivate, PQ 10, PQ 67, kationisch modifizierten Guarderivate wie Dehyquart^{®} Guar N, Guar Hydroxypropyltrimonium Chlorid, kationischen Homo- oder Copolymeren auf der Basis von Acrylamid, kationischen Homo- oder Copolymeren auf der Basis von Vinylpyrrolidon, kationischen Homo- oder Copolymeren auf der Basis von quaternisiertem Vinylimidazol und kationischen Homo- oder Copolymeren auf der Basis von Methacrylaten.

Geeignete kationische Polymere sind beispielsweise die quaternierte Hydroxyethylcellulose, die auch unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Proteinhydrolysate, Polypeptide und Aminsäuren wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol. Insbesondere geeignete kationische Polymere sind Polyquaternium-68, erhältlich als Luviquat^{®} Supreme AT 1, oder Polyquaternium-11, erhältlich als Luviquat^{®} PQ 11 AT 1.

Die kationischen Polymere sind in den Haarbehandlungsmitteln vorzugsweise in Mengen von 0,02 bis 5 Gew.-%, vorzugsweise 0,05 bis 3 Gew.-% und besonders bevorzugt in Mengen von 0,1 bis 2 Gew.-% enthalten.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/ Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methyl-benzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt oder Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2.4-dichlorphenyl)r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

### Weitere Zusatzstoffe

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Betracht.

### Proteinhydrolysate

Falls gewünscht können weitere aus dem Stand der Technik bekannte Proteinhydrolysate eingesetzt werden, beispielsweise auf Basis von Keratin wie das im Handel erhältliche Nutrilan^{®} Keratin W PP oder auf Basis von Weizen wie Gluadin^{®} WLM Benz, Gluadin^{®} WK oder Gluadin^{®} WP. Es ist auch, möglich geringe Mengen an freien Aminosäuren wie Lysin oder Arginin zuzugeben.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Benzoate, Phenoxyethanol, Formaldehydlösung, Parabene, Pentandiol, Sorbinsäure, Lävulinsäure und Arachidonsäure sowie die unter der Bezeichnung Surfacine^{®} bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.1.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Kosmetische Mittel

Besonders bevorzugt werden im Sinne der vorliegenden Erfindung kosmetische Mittel, die außer den erfindungsgemäßen Keratinhydrolysaten zusätzlich kationische Polymere oder kationische bzw. pseudokationische Tenside enthalten.

Gemäß einer Ausführungsform enthält das kosmetische Mittel zur konditionierenden Haarbehandlung neben den erfindungsgemäßen Keratinhydrolysaten, kationische Polymere der schon beschriebenen Art sowie optional zusätzlich Emulgatoren, Hydrotrope oder weitere übliche Inhaltsstoffe, wobei zur Ergänzung auf 100 Gew.-% Wasser enthalten ist. Konditionierende Haarmittels in Form eines Serums haben vorzugsweise derartige Zusammensetzungen.

Gemäß einer weiteren Ausführungsform enthält das kosmetische Mittel für die konditionierende Haarbehandlung (auch Haarbehandlungsmittel genannt) in Kombination mit den erfindungsgemäßen Keratinhydrolysaten
- kationische Tenside
- Wachse, vorzugsweise Glyceridester und/oder Fettalkohol und
- Wasser sowie
- optional mindestens ein kationisches Polymer und/oder
- optional nichtionische Tenside.

Innerhalb dieser Ausführungsform der konditionierenden Haarbehandlungsmittel sind bevorzugt enthalten
0,002 bis 0,4 Gew.-% - bezogen auf Aktivsubstanzgehalt- erfindungsgemäßes Keratinhydrolysat,
0,1 bis 15 Gew.-% kationische Tenside,
0,5 bis 15 Gew.-% eines Wachses, vorzugsweise Triglyceridester und/oder Fettalkohol und
0,0 bis 5 Gew.-% mindestens eines kationischen Polymers und/oder
0,0 bis 15 Gew.-% nichtionische Tenside enthalten, wobei optional weitere übliche Inhaltsstoffe enthalten sein können und zur Ergänzung auf 100 Gew.-% Wasser.

Ein anderer Gegenstand der vorliegenden Erfindung betrifft kosmetische Mittel zur Reinigung von Haut und/oder Haar, dadurch gekennzeichnet, dass sie die erfindungsgemäßen Keratinhydrolysate sowie zusätzlich anionische Tenside und amphotere bzw. zwitterionische Tenside enthalten.

Hierunter sind alle kosmetischen Reinigungsmittel zu verstehen, die zur Reinigung und Pflege der Haut gedacht sind, vorzugsweise Duschgele, Duschbäder, Duschöle, Schaumbäder oder Handdesinfektions-Lotionen.

Gemäß dieser Ausführungsform enthält das kosmetische Mittel in Kombination mit den erfindungsgemäßen Keratinhydrolysaten zusätzlich anionische Tenside, amphotere bzw. zwitterionische Tenside sowie optional nichtionische Tenside, optional ein kationisches Polymer und/oder optional Perlglanzwachs, wobei optional weitere übliche Inhaltsstoffe enthalten sein können und zur Ergänzung auf 100 Gew.-% Wasser.

Innerhalb dieser Ausführungsform sind bevorzugt
0,002 bis 0,4 Gew.-% - bezogen auf Aktivsubstanzgehalt - erfindungsgemäßes Keratinhydrolysat, 8 bis 15 Gew.-% anionische Tenside,
0,5 bis 5 Gew.-% amphotere bzw. zwitterionische Tenside,
0,0 bis 5 Gew.-% mindestens ein kationisches Polymer,
0,0 bis 3 Gew.-% nichtionische Tenside, und
0,0 bis 2 Gew.-% Perlglanzwachse sowie optional weitere in Hautbehandlungsmittel übliche Inhaltsstoffe und zur Ergänzung auf 100 Gew.-% Wasser, enthalten.

Beispiele für geeignete Kombinationen der kosmetischen Mittel sind im folgenden Beispielsteil zu finden.

### Beispiele

### A) Herstellbeispiele der Keratinhydrolysate

### Erfindungsgemäßes Beispiel 1 niedermolekulares Keratinhydrolysat

100 g Wasser wurden vorgelegt und mit einer Base auf pH 10 eingestellt. Anschließend wurden 10 g Keratinwolle eingerührt und durch Zugabe 5 g einer 30%igen Wasserstoffperoxid (H₂O₂) - Lösung die oxidative Vorbehandlung der Wolle gestartet (Dauer: ~ 10 Stunden). Danach wurde der pH-Wert auf pH 8,5 eingestellt und zunächst 0,15 g Protease (aus *Bacillus licheniformis*; z.B. Alcalase^{®} von Novozymes) zugegeben. Nach vollständiger Hydrolyse mit diesem Enzym wurde der pH-Wert auf pH 7 gesenkt und anschließend 0,15 g Exopeptidase (aus *Aspergillus oryzae,* z.B. Flavourzyme^{®} von Novozymes) zugegeben und ebenfalls wieder vollständig hydrolysiert. Abschließend wurden die verbleibenden nicht wasserlöslichen Wollreste mittels Filtration abgetrennt und das erhaltene Produkt zur Stabilisierung auf pH 4,2 eingestellt.

Durch Aufkonzentrieren wurde das Keratinhydrolysat als 20 gew.-%ige wässrige Lösung formuliert.

### Vergleichsbeispiel Keratinhydrolysat (nicht erfindungsgemäß)

100 g Wasser wurden vorgelegt und mit einer Base auf ~ pH 9,4 eingestellt. Anschließend wurden 3,75 g Keratinwolle eingerührt und durch Zugabe von 1,9 g einer 30%igen Wasserstoffperoxid (H₂O₂) -Lösung die oxidative Vorbehandlung der Wolle gestartet (Dauer: ~ 10 Stunden). Anschließend wurden 0,10 g einer Protease (z.B. aus *Bacillus licheniformis*; Alcalase von Novozymes) zugegeben. Nach vollständiger Hydrolyse wurden die verbleibenden nicht wasserlöslichen Wollreste mittels Filtration abgetrennt und das erhaltene Produkt zur Stabilisierung auf pH 4,2 eingestellt.

Durch Aufkonzentrieren wurde das Keratinhydrolysat als 20 gew.-%ige wässrige Lösung formuliert.

### Molekulargewicht der Keratinhydrolysate:

Das Molekulargewicht sowie die Verteilung der Molmassen wurde bestimmt mittels Größenausschlußchromatografie "Size Exclusion Chromatography (SEC)", eine Flüssigchromatografie, bei der die Moleküle gelöster Stoffe aufgrund ihrer Größe getrennt werden. SEC ist eine für Proteine gängige Methode (siehe auch Wikipedia Artikel Gel-Permeations-Chromatografphie vom 17.06.2016). Gewählt wurde die Chromatographiesäule "Superdex Peptide" von GE Healthcare Life Sciences zur hochauflösenden Trennung von Proteinen und Peptiden, die besonders gut für die Bestimmung von Proteinfragmenten mit den Molekulargewichten im Bereich von 100 bis 7000 geeignet ist.

| | **Vergleichsbeispiel** | **Beispiel 1** |
|---|---|---|
| Mittleres Molekulargewicht | ~ 3600 Da | ~ 2000 Da |
| Anteile < 500 Da | < 15 % | ~ 28 % |
| Anteile < 1.000 Da | ~ 25 % | ~ 38 % |
| Anteile < 3.500 Da | ~ 55 % | ~ 70 % |
| Anteile > 3.500 Da | ~ 45 % | ~ 30 % |
| Anteile > 20.000 Da | ~ 1 % | < 0,01 % |

Die % Angabe bezieht sich auf die Gesamtzahl der Proteinfragmente, d.h. für Beispiel 1 beispielsweise, dass 38 % aller Proteinfragmente ein Molekulargewicht unter 1.000 Da aufweisen.

### B) Anwendungsbeispiele

### B1) Zug-Dehnungsversuch ("Tensile Strength Method") zur Analyse des Einflusses der Keratinhydrolysate auf die Festigkeit und die Elastizität der Haare

Mit der Tensile Strength Methode kann die mechanische Stabilität des Haares, vor allen Dingen der Cortex-Region bestimmt werden. Hierzu wird die Kraft gemessen, die notwendig ist, ein einzelnes Haar in die Länge zu ziehen. Die gemessenen Kräfte werden anschließend auf den Querschnitt der Haare normalisiert und man erhält ein sogenanntes Spannungs-Dehnungs-Diagramm mit Hilfen dessen man die Eigenschaften des Haares ermitteln kann.

Es wurde ein vollautomatisiertes Dia-Stron MTT680 der Firma Diastron, UK mit einer Laser-Messvorrichtung zu einer 4-Punkt Querschnittsmessung des Haares und einer Messvorrichtung für die Kraftmessung verwendet.

Getestet wurden je 3 cm lange einzelne Haare, die in Metallplatten eingespannt waren. Nach der Bestimmung des Haarquerschnittes wurde das in Metallplatten eingespannte Haar vor der Messung 60 min in entmineralisiertem Wasser bedeckt. Auch während der Messung verblieben die Haarsträhnen im Wasser.

Getestet wurde eine 5 %ige Keratinhydrolysat-Lösung nach Beispiel 1 bzw. Vergleichsbeispiel in Wasser.

Statistische Signifikanz (p=0,05) berechnet nach dem Tukey's HSD Test

### Ergebnisse:

| | **Vergleichsbeispiel** | **Beispiel 1** |
|---|---|---|
| Zugfestigkeit vs. Placebo | + 5,8 % | + 18,7 % |
| Elastizität vs. Placebo | -1,47·10⁷ N/mm² | 5,23·10⁷ N/mm² |

Das mit dem erfindungsgemäßen Keratinhydrolysat behandelte Haar zeigt eine deutlich bessere Zugfestigkeit und Elastizität als das Haar ohne Behandlung ("Placebo"). Es zeigt aber auch deutlich bessere Ergebnisse als Haare behandelt mit dem Vergleichsbeispiel, beispielsweise eine 3 Mal bessere Zugfestigkeit und bessere Elastizität, was auf eine Stärkung des Haares von innen heraus deutet.

### B2) Methode zur Bestimmung von Haarbruch (hair breakage test):

Die Widerstandskraft des Haares gegen mechanische Beanspruchung wurde mit dem sog. "hair breakage test" bestimmt. Dazu wurden die Haarsträhnen mit einem Keratinhydrolysat enthaltenen Conditioner (Zusammensetzung siehe unten) behandelt. Nach 3 Minuten Einwirkung wurde 1 Minute abgespült und der Vorgang 1 Mal wiederholt. Anschließend wurden die Haarsträhnen auf ein Gitter gelegt und für 1 Stunde mit dem Fön getrocknet. Danach wurden die Haarsträhnen 50.000 Mal mit einer Kämmmaschine gekämmt. Die abgebrochenen Haarbruchstücke wurden gewogen und in das Verhältnis zu dem Gewicht der Haarsträhne gesetzt.

Statistische Signifikanz (p=0,05) berechnet nach dem Tukey's HSD Test Zusammensetzung des getesteten Conditioners; die Angaben sind Gewichts%:

| | **INCI** | **Placebo** | **Vergleichsbeispiel** | **Erfindungsgemäßes Beispiel** |
|---|---|---|---|---|
| Lanette^{®} O | Cetyl Stearyl Alcohol | 4.5 | 4.5 | 4.5 |
| Myritol^{®} 312 | Caprylic/Capric Triglyceride | 2 | 2 | 2 |
| Dehyquart^{®} A CA | Cetyl Trimethyl Ammonium Chloride | 2 | 2 | 2 |
| Dehyquart^{®} F 75 | Distearoylethyl Hydroxyethylmonium Methosulfate (and) Cetearyl Alcohol | 1.4 | 1.4 | 1.4 |
| **Vergleichsbeispiel 1** | | | **1** | |
| **Beispiel 1** | | | | **1** |
| Natriumbenzoat | | 0.3 | 0.3 | 0.3 |
| Parfum | | 0.3 | 0.3 | 0.3 |
| Wasser | | 89.5 | 88.5 | 88.5 |

**Ergebnisse:**

| | **Vergleichsbeispiel** | **Beispiel 1** |
|---|---|---|
| Haarbruch vs. Placebo | Keine Verbesserung | 50 % weniger Haarbruch |

Das mit dem erfindungsgemäßen Keratinhydrolysat behandelte Haar zeigt deutlich weniger Haarbruch als das Haar ohne Keratinhydrolysat im getesteten Conditioner ("Placebo") und als die Haarsträhnen behandelt mit dem Vergleichsbeispiel enthaltenden Conditioner. Das Ergebnis zeigt die stärkende Wirkung der erfindungsgemäßen Keratinhydrolysate für das Innere des Haares.

### B3) Conditioner mit Proteinhydrolysat

Es wurden Conditioner mit folgender Zusammensetzung hergestellt (Angaben in Gew.-%):

| | **INCI** | **Conditioner ohne Keratinhydrolysat ("Placebo")** | **Conditioner Vergleich** | **Conditioner erfindungsgemäß** |
|---|---|---|---|---|
| Cosmedia^{®} Triple C | Polyquaternium 37(and) Dicaprylylcarbonat(and) Lauryl Glucoside | 0.40 | 0.40 | 0.40 |
| Dehyquart^{®} F 75/ Conditioning | Distearoylethyl Hydroxyethylmonium Methosulfate (and) Cetearyl Alcohol | 2.50 | 2.50 | 2.50 |
| Lanette^{®} O | Cetyl stearyl alcohol | 3.50 | 3.50 | 3.50 |
| Cutina^{®} HVG | Hydrogenated Vegetable Glycerides | 1.00 | 1.00 | 1.00 |
| Cetiol^{®} C 5 | Coco Caprylate | 2.00 | 2.00 | 2.00 |
| Cetiol^{®} SB 45 | Butyrospermum Parkii Butter | 0.70 | 0.70 | 0.70 |
| Lipofructyl^{®} Argan LS 9779 | Argania Spinosa Kernel Oil | 0.20 | 0.20 | 0.20 |
| Wasser | | 85.90 | 83.90 | 83.90 |
| Dehyquart^{®} A-CA | Cetyl Trimethyl Ammonium Chloride | 3.00 | 3.00 | 3.00 |
| **Vergleichsbeispiel** | | **0** | **2.00** | |
| **Beispiel 1** | | **0** | | **2.00** |
| Parfum | | 0.30 | 0.30 | 0.30 |
| Natriumbenzoat | | 0.50 | 0.50 | 0.50 |
| Zitronensäure | | q.s. | q.s. | q.s. |

Die Conditioner wurden einem sensorischen Test unterzogen. An dem sensorischen Test nehmen standardmäßig 11 Probanden teil, die nacheinander in einem klimatisierten Raum (T = 22°C, rel. Luftfeuchte = 40%) die Bewertung von mit Conditionern behandelten Haarsträhnen im paarweisen Vergleich Conditioner mit Vergleichs-Keratinhydrolysat gegen Conditioner Placebo und Conditioner mit erfindungsgemäßem Keratinhydrolysat gegen Placebo im nassen und im trockenen Zustand durchführen. Die zu bewertenden Attribute wie Kämmbarkeit der Haarsträhnen, Glanz, Volumen sind vorher mit den Probanden intensiv trainiert worden. Zur statistischen Auswertung wird ein dem Versuchsprotokoll angepasste Berechnung, der Wilcoxon-Test, herangezogen, um Signifikanzen in den Profilen für die Produkte zu bestimmen.

Nach drei Minuten Einwirkzeit beurteilten 10 von 11 Probanden den pflegenden Charakter des Conditioners mit dem erfindungsgemäßen Keratinhydrolysat als besser als der des Placebo-Conditioners. Außerdem bewertete weit über die Hälfte der Probanden das Volumen der zu untersuchenden Haarsträhne als voller und der Conditioner mit dem erfindungsgemäßen Keratinhydrolysat wurde von den Probanden besser akzeptiert. Nach 15 Minuten Einwirkzeit beurteilten die Probanden das Volumen der Haarsträhne, die mit dem Conditioner mit dem erfindungsgemäßen Proteinhydrolysat behandelt wurden, als besser als das mit dem Placebo-Conditioner behandelte Haar. Der Conditioner mit dem Keratinhydrolysat nach Vergleichsbeispiel zeigte im Volumen keinen Unterschied zum Placebo-Conditioner.

### B4) Verbesserte sensorische Eigenschaften für einen sog. Leave-in - Serum - Conditioner für trockenes Haar

| | **INCI** | **ohne Keratinhydrolysat ("Placebo")** | **Vergleich** | **Erfindungsgemäß** |
|---|---|---|---|---|
| Wasser | | 93.53 | 91.53 | 91.53 |
| **Vergleichsbeispiel** | | | **2.00** | |
| **Beispiel 1** | | | | **2.00** |
| Melhydran^{®} LS 9876 | Aqua(and)Butylene Glycol(and)Mel Extract (and) Glycerin (and) Urea | 1.00 | 1.00 | 1.00 |
| D-Panthenol^{®} 75 W | Panthenol | 0.67 | 0.67 | 0.67 |
| Glycerin | Glycerin | 3.00 | 3.00 | 3.00 |
| Natriumbenzoat | | 0.5 | 0.5 | 0.5 |
| Eumulgin^{®} CO 40 | PEG-40 Hydrogenated Castor Oil | 0.5 | 0.5 | 0.5 |
| Copherol^{®} 1250 C | Tocopheryl Acetate | 0.5 | 0.5 | 0.5 |
| Parfum | | 0.3 | 0.3 | 0.3 |
| Zitronensäure | | q.s | q.s | q.s |

Bei der sensorischen Beurteilung wurde analog B3) verfahren. Der Leave-in Serum-Conditionermit dem erfindungsgemäßen Proteinhydrolysat wurde von deutlich mehr als die Hälfte der Probanden hinsichtlich Glanz als auch die Geschmeidigkeit der Haarsträhne besser beurteilt gegenüber dem Placebo-Conditioner. Für die Formulierung mit dem Keratinhydrolysat nach Vergleichsbeispiel wurde dieser Effekt nicht nachgewiesen.

### B5) Anwendung in Haarshampoos

Folgende Formulierungen wurden hergestellt. Die Angaben sind Gew.-%:
Shampoo 1

| **Inhaltstoff** | **INCI** | **%** |
|---|---|---|
| Texapon^{®} N 70 | Sodium Laureth Sulfate | 14.30 |
| Dehyton^{®} PK 45 | Cocamidopropyl Betaine | 5.40 |
| Dehyquart^{®} Guar N | Guar Hydroxypropyltrimonium Chloride | 0.20 |
| Lamesoft^{®} Care | PEG-4 Distearyl Ether (and) Sodium Laureth Sulfate (and) Distearyl Ether (and) Dicaprylyl Ether | 3.00 |
| Dehydol^{®} LS 2 Deo N | Laureth-2 | 1.00 |
| Water, demin. | Aqua | 74.60 |
| Beispiel 1 | | 0.50 |
| Sodium Chloride | Sodium Chloride | 0.50 |
| Sodium Benzoate | Sodium Benzoate | 0.50 |
| Citric Acid | Citric Acid | qs |

Shampoo 2

| **Inhaltstoff** | **INCI** | **%** |
|---|---|---|
| Texapon^{®} N 70 | Sodium Laureth Sulfate | 14.30 |
| Dehyton^{®} PK 45 | Cocamidopropyl Betaine | 5.40 |
| Water, demin. | Aqua | 77.30 |
| Beispiel 1 | | 0.50 |
| Sodium Chloride | Sodium Chloride | 2.00 |
| Sodium Benzoate | Sodium Benzoate | 0.50 |
| Citric Acid | Citric Acid | qs |

Shampoo 3

| **Inhaltstoff** | **INCI** | **%** |
|---|---|---|
| Texapon^{®} N 70 | Sodium Laureth Sulfate | 14.30 |
| Dehyton^{®} PK 45 | Cocamidopropyl Betaine | 5.40 |
| Dehyquart^{®} Guar N | Guar Hydroxypropyltrimonium Chloride | 0.20 |
| Water, demin. | Aqua | 76.70 |
| Beispiel 1 | | 1.00 |
| Sodium Chloride | Sodium Chloride | 1.60 |
| Perfume Cotton Touch | Parfum | 0.30 |
| Sodium Benzoate | Sodium Benzoate | 0.50 |
| Citric Acid | Citric Acid | qs |

Kämmversuche auf trockenen Haar zeigten eine Reduzierung der benötigten Kämmarbeit von 20 % im Vergleich zum Placebo-Shampoo ohne erfindungsgemäßes Keratinhydrolysat.
Shampoo 4 "Extra Care"

| **Inhaltstoff** | **INCI** | **%** |
|---|---|---|
| Water | Water | 68.12 |
| Dehyquart^{®} Guar N | Guar Hydroxypropyltrimonium Chloride | 0.20 |
| Dehyton^{®} PK 45 | Cocamidopropyl Betaine | 2.70 |
| Luviquat^{®} UltraCare | Polyquaternium-44 | 1.15 |
| Texapon^{®} N 70 | Sodium Laureth Sulfate | 12.90 |
| Plantapon^{®} LC 7 | Laureth-7 Citrate | 1.00 |
| Plantacare^{®} 818 UP | Coco-Glucoside | 0.40 |
| Lamesoft^{®} PO 65 | Coco-Glucoside, Glyceryl Oleate | 5.00 |
| Cetiol^{®} LDO | Dicaprylyl Ether, Lauryl Alcohol | 0.70 |
| Beispiel 1 | | 2.00 |
| D-Panthenol 75 W | Panthenol | 1.33 |
| Euperlan^{®} PK 710 Benz | Glycol Distearate, Sodium Laureth Sulfate, Cocamide MEA | 3.00 |
| Sodium Benzoate | Sodium Benzoate | 0.50 |
| Perfume | Parfum | qs |
| Magnesium Chloride Hexahydrate | Magnesium Chloride | 1.00 |
| Citric Acid (50% solution) | Citric Acid | qs |
| Sodium Chloride | Sodium Chloride | qs |

Shampoo 5 Thickening Shampoo

| **Inhaltstoff** | **INCI** | **%** |
|---|---|---|
| Texapon^{®} N 70 | Sodium Laureth Sulfate | 14.30 |
| Dehyton^{®} PK 45 | Cocamidopropyl Betaine | 5.40 |
| Plantapon^{®} LC 7 | Laureth-7 Citrate | 2.00 |
| Gluadin^{®} WQ PP | Laurdimonium Hydroxypropyl Hydrolyzed Wheat Protein | 0.50 |
| Beispiel 1 | | 2.00 |
| D-Panthenol 75 W | Panthenol | 1.33 |
| Euperlan^{®} PK 710 Benz | Glycol Distearate, Sodium Laureth Sulfate, Cocamide MEA | 3.00 |
| Perfume | Parfum | qs |
| Water | Water | 68.97 |
| Sodium Benzoate | Sodium Benzoate | 0.50 |
| Magnesium Chloride Hexahydrate | Magnesium Chloride | 1.00 |
| Dehydol LS 2 Deo N | Laureth-2 | 1.00 |
| Citric Acid (50% solution) | Citric Acid | qs |
| Sodium Chloride | Sodium Chloride | qs |

### B6) Conditioner als Gel

Conditioning Gel

| **Inhaltstoff** | **INCI** | **%** |
|---|---|---|
| Water, demin. | Aqua | 80.90 |
| Beispiel 1 | | 1.50 |
| Ethanol | Alcohol | 15.00 |
| Dehyquart^{®} Guar HP | Guar Hydroxypropyltrimonium Chloride | 1.50 |
| Citric Acid | Citric Acid | 0.30 |
| Luviquat^{®} Supreme AT 1 | Polyquaternium-68 | 2.00 |
| Perfume Cotton Touch | Parfum | 0.30 |

### B7) Styling Mousse

| **Inhaltstoff** | **INCI** | **%** |
|---|---|---|
| Water, demin. | Aqua | 82.70 |
| Beispiel 1 | | 2.00 |
| Luviquat^{®} Supreme AT 1 | Polyquaternium-68 | 7.50 |
| Luviquat^{®} PQ 11 AT 1 | Polyquaternium-11 | 7.50 |
| Cremophor^{®} A 25 | Ceteareth-25 | 0.50 |
| Perfume Cotton Touch | Parfum | 0.10 |
| Sodium Benzoate | Sodium Benzoate | 0.30 |
| Citric Acid (20% solution) | Citric Acid | 1.40 |

### B8) Conditioner für Naturkosmetik

| **Inhaltstoff** | **INCI** | **%** |
|---|---|---|
| Plantaquat^{®} NC | Cetearyl Alcohol (and) Lecithin (and) Sodium Cetearyl Sulfate (and) Olus Oil [EU] | 9.0 |
| Lanette^{®} O | Cetearyl Alcohol | 2.0 |
| Cetiol^{®} C5 | Coco-Caprylate | 2.0 |
| Cegesoft^{®} SB | Butyspermum Parkii Butter | 2.0 |
| Beispiel 1 | | 2.0 |
| Sodium Benzoate | Sodium Benzoate | 0.5 |
| Perfume | Perfume | q.s. |
| Water | Water | add 100 |

### B9) Hair and Body Wash

**Hair und Body Wash -Reiniunsmittel 1 für Naturkosmetik**

| **Inhaltstoff** | **INCI** | **%** |
|---|---|---|
| Water, demin. | Water | 68.20 |
| Sulfopon^{®} 1216 G | Sodium Coco-Sulfate | 8.00 |
| Plantacare^{®} 818 UP | Coco-Glucoside | 13.30 |
| Euperlan^{®} GREEN | Lauryl Glucoside (and) Stearyl Citrate | 2.00 |
| Lamesoft^{®} PO 65 | Coco-Glucoside (and) Glyceryl Oleate | 2.00 |
| Beispiel 1 | | 0.50 |
| Sodium Benzoate | Sodium Benzoate | 0.50 |
| Perfume | Perfume | q.s. |
| Apfelsaft 100% Direktsaft | Pyrus Malus (Apple) Juice | 3.00 |
| Citric Acid (50%) | Citric Acid | 1.20 |
| Sodium Chloride | Sodium Chloride | 1.30 |

**Hair und Body Wash -Reinigungsmittel 2 für Naturkosmetik**

| **Inhaltstoff** | **INCI** | **%** |
|---|---|---|
| Water, demin. | Water | 70.10 |
| Sulfopon^{®} 1216 G | Sodium Coco-Sulfate | 8.00 |
| Plantacare^{®} 818 UP | Coco-Glucoside | 13.30 |
| Lamesoft^{®} PO 65 | Coco-Glucoside (and) Glyceryl Oleate | 2.00 |
| Beispiel 1 | | 0.50 |
| Sodium Benzoate | Sodium Benzoate | 0.50 |
| Perfume | Perfume | q.s. |
| Apfelsaft 100% Direktsaft | Pyrus Malus (Apple) Juice | 3.00 |
| Citric Acid (50%) | Citric Acid | 1.20 |
| Sodium Chloride | Sodium Chloride | 1.30 |

## Patentansprüche

1. Verfahren zur Herstellung von Keratinhydrolysaten, bei denen mindestens 30 % der enthaltenen Proteinfragmente ein Molekulargewicht unter 1000 Da und mindestens 15% ein Molekulargewicht unter 500 Da aufweisen, wobei zunächst a) Keratin oxidativ vorbehandelt und b) in Anwesenheit mindestens einer Endopeptidase enzymatisch hydrolysiert wird, **dadurch gekennzeichnet, dass** anschließend c) in Anwesenheit von mindestens einer Exopeptidase ein weiteres Mal enzymatisch hydrolysiert wird.

2. Verfahren zur Herstellung von Keratinhydrolysaten nach Anspruch 1, **dadurch gekennzeichnet, dass** b) enzymatisch hydrolysiert wird in Anwesenheit von Endopeptidasen ausgewählt aus den Bacillusstämmen, insbesondere von Bacillus licheniformis.

3. Verfahren zur Herstellung von Keratinhydrolysaten nach Anspruch 1 oder 2, **dadurch gekennzeichnet, dass** c) enzymatisch hydrolysiert wird in Anwesenheit von Exopeptidasen zugänglich aus Aspergilllus oryzae.

4. Verfahren zur Herstellung von Keratinhydrolysaten nach Anspruch 1, **dadurch gekennzeichnet, dass** mindestens 35% der enthaltenen Proteinfragmente ein Molekulargewicht unter 1000 Da aufweisen.

5. Verfahren zur Herstellung von Keratinhydrolysaten nach Anspruch 1, **dadurch gekennzeichnet, dass** mindestens 50 % der enthaltenen Proteinfragmente mit einem Molekulargewicht unter 1000 Da ein Molekulargewicht über 100 Da aufweisen.

6. Verfahren zur Herstellung von Keratinhydrolysaten nach Anspruch 1, **dadurch gekennzeichnet, dass** mindestens 50% der enthaltenen Proteinfragmente mit einem Molekulargewicht unter 500 ein Molekulargewicht über 100 Da aufweisen.

7. Verwendung der Keratinhydrolysate nach Anspruch 1 zur Verbesserung mindestens einer der Eigenschaften in Haarbehandlungsmitteln:
- Verbesserung der inneren Haarstruktur
- Schutz vor haarschädigenden Umwelteinflüssen
- Reparatur geschädigter Haare
- Zugfestigkeit von menschlichen Haaren
- Stabilisierung des Feuchtigkeitshaushaltes von menschlichen Haaren
- Kämmbarkeit von menschlichen Haaren
- Haarbruch von menschlichen Haaren
- Restrukturierbarkeit von menschlichen Haaren
- Verringerung der Elastizitätsabnahme von menschlichen Haaren

8. Verwendung der Keratinhydrolysate nach Anspruch 1 als Conditioner in Haarbehandlungsmitteln.

9. Verwendung der Keratinhydrolysate nach Anspruch 1 zur Verbesserung der Sensorik in kosmetischen Mitteln zur Reinigung von Haut und/oder Haar, vorzugsweise der Schaumsensorik und insbesondere in Duschbädern oder Duschgelen.

10. Kosmetisches Mittel enthaltend Keratinhydrolysate nach Anspruch 1, **dadurch gekennzeichnet, dass** sie Keratinhydrolysate in Mengen von 0,002 bis 0,4 Gew.-% - bezogen auf Aktivsubstanzgehalt an Keratinhydrolysat - enthalten.

11. Kosmetische Mittel für die konditionierende Haarbehandlung, **dadurch gekennzeichnet, dass** sie Keratinhydrolysate nach Anspruch 1 und/oder 10 sowie zusätzlich kationische Polymere oder kationische bzw. pseudokationische Tenside enthalten.

12. Kosmetisches Mittel zur Reinigung von Haut und/oder Haar, **dadurch gekennzeichnet, dass** sie Keratinhydrolysate nach Anspruch 1 und/oder 10 sowie zusätzlich anionische Tenside und amphotere bzw. zwitterionische Tenside enthalten.

## Claims

1. A method for preparing keratin hydrolyzates, in which at least 30% of the protein fragments present have a molecular weight of less than 1000 Da and at least 15% have a molecular weight of less than 500 Da, wherein firstly a) keratin is oxidatively pretreated and b) enzymatically hydrolyzed in the presence of at least one endopeptidase, wherein subsequently c) enzymatic hydrolysis is carried out once again in the presence of at least one exopeptidase.

2. The method for preparing keratin hydrolyzates according to claim 1, wherein b) enzymatic hydrolysis is carried out in the presence of endopeptidases selected from Bacillus strains, especially from Bacillus licheniformis.

3. The method for preparing keratin hydrolyzates according to claim 1 or 2, wherein c) enzymatic hydrolysis is carried out in the presence of exopeptidases obtainable from Aspergillus oryzae.

4. The method for preparing keratin hydrolyzates according to claim 1, wherein at least 35% of the protein fragments present have a molecular weight of less than 1000 Da.

5. The method for preparing keratin hydrolyzates according to claim 1, wherein at least 50% of the protein fragments present having a molecular weight of less than 1000 Da have a molecular weight of greater than 100 Da.

6. The method for preparing keratin hydrolyzates according to claim 1, wherein at least 50% of the protein fragments present having a molecular weight of less than 500 Da have a molecular weight of greater than 100 Da.

7. The use of the keratin hydrolyzates according to claim 1 for improving at least one of the properties in hair treatment compositions:
- improving the internal hair structure
- protecting against environmental influences that damage the hair
- repairing damaged hair
- tensile strength of human hair
- stabilizing the moisture balance of human hair
- combability of human hair
- breakage of human hair
- restructurability of human hair
- reducing the loss of elasticity of human hair.

8. The use of the keratin hydrolyzates according to claim 1 as conditioner in hair treatment compositions.

9. The use of the keratin hydrolyzates according to claim 1 for improving the sensory feel in cosmetic compositions for cleansing skin and/or hair, preferably the sensory feel of foam and especially in body washes or shower gels.

10. A cosmetic composition comprising keratin hydrolyzates according to claim 1, wherein said composition comprises keratin hydrolyzates in amounts of 0.002 to 0.4 wt% based on the active substance content of keratin hydrolyzate.

11. The cosmetic compositions for conditioning hair treatment, wherein said compositions comprise keratin hydrolyzates according to claim 1 and/or 10 and also additional cationic polymers or cationic or pseudo-cationic surfactants.

12. The cosmetic composition for cleansing skin and/or hair, wherein said composition comprises keratin hydrolyzates according to claim 1 and/or 10 and also additional anionic surfactants and amphoteric or zwitterionic surfactants.

## Revendications

1. Procédé de fabrication d'hydrolysats de kératine, dans lesquels au moins 30 % des fragments de protéine obtenus présentent une masse moléculaire inférieure à 1 000 Da et au moins 15 % une masse moléculaire inférieure à 500 Da, dans lequel d'abord a) on soumet la kératine à un prétraitement oxydatif et b) on procède à une hydrolyse enzymatique en présence d'au moins une endopeptidase, **caractérisé en ce qu'**ensuite c) on procède une fois de plus à une hydrolyse enzymatique en présence d'au moins une exopeptidase.

2. Procédé de fabrication d'hydrolysats de kératine selon la revendication 1, **caractérisé en ce que** b) on procède à une hydrolyse enzymatique en présence d'endopeptidases choisies parmi les souches de Bacillus, en particulier Bacillus licheniformis.

3. Procédé de fabrication d'hydrolysats de kératine selon la revendication 1 ou 2, **caractérisé en ce que** c) on procède à une hydrolyse enzymatique en présence d'exopeptidases pouvant être obtenues à partir d'Aspergillus oryzae.

4. Procédé de fabrication d'hydrolysats de kératine selon la revendication 1, **caractérisé en ce qu'**au moins 35 % des fragments de protéine obtenus présentent une masse moléculaire inférieure à 1 000 Da.

5. Procédé de fabrication d'hydrolysats de kératine selon la revendication 1, **caractérisé en ce qu'**au moins 50 % des fragments de protéine obtenus ayant une masse moléculaire inférieure à 1 000 Da présentent une masse moléculaire supérieure à 100 Da.

6. Procédé de fabrication d'hydrolysats de kératine selon la revendication 1, **caractérisé en ce qu'**au moins 50 % des fragments de protéine obtenus ayant une masse moléculaire inférieure à 500 présentent une masse moléculaire supérieure à 100 Da.

7. Utilisation des hydrolysats de kératine selon la revendication 1 pour améliorer au moins l'une des propriétés d'agents de traitement des cheveux :
- Amélioration de la structure interne des cheveux
- Protection des influences environnementales nocives pour les cheveux
- Réparation des cheveux endommagés
- Résistance à la traction des cheveux humains
- Stabilisation de l'hydratation des cheveux humains
- Peignabilité des cheveux humains
- Cassure des cheveux humains
- Aptitude à la restructuration des cheveux humains
- Diminution de la perte d'élasticité des cheveux humains.

8. Utilisation des hydrolysats de kératine selon la revendication 1 comme conditionnant dans les agents de traitement des cheveux.

9. Utilisation des hydrolysats de kératine selon la revendication 1 pour améliorer les propriétés organoleptiques des produits cosmétiques destinés au nettoyage de la peau et/ou des cheveux, de préférence les caractéristiques organoleptiques de la mousse et en particulier dans les bains-douches ou les gels-douches.

10. Produits cosmétiques contenant des hydrolysats de kératine selon la revendication 1, **caractérisés en ce qu'**ils contiennent des hydrolysats de kératine en des quantités de 0,002 à 0,4 % en poids - par rapport à la quantité de matière active dans l'hydrolysat de kératine.

11. Produits cosmétiques pour le traitement conditionnant des cheveux, **caractérisés en ce qu'**ils contiennent des hydrolysats de kératine selon les revendications 1 et/ou 10, ainsi qu'en outre des polymères cationiques ou des tensioactifs cationiques ou pseudo-cationiques.

12. Produits cosmétiques pour le nettoyage de la peau et/ou des cheveux, **caractérisés en ce qu'**ils contiennent des hydrolysats de kératine selon les revendications 1 et/ou 10 ainsi qu'en outre des tensioactifs anioniques et des tensioactifs amphotères ou zwitterioniques.
